(19) Europäisches Patentamt / European Patent Office / Office européen des brevets

(11) **EP 4 356 833 A1**

(12) **EUROPEAN PATENT APPLICATION**
published in accordance with Art. 153(4) EPC

(43) Date of publication:
**24.04.2024 Bulletin 2024/17**

(21) Application number: **21946142.3**

(22) Date of filing: **17.06.2021**

(51) International Patent Classification (IPC):
**A61B 5/16** (2006.01)    **A61B 5/00** (2006.01)

(52) Cooperative Patent Classification (CPC):
**A61B 5/00; A61B 5/16; G06N 20/00; G16H 20/70; G16H 50/50; G16H 50/70**

(86) International application number:
**PCT/KR2021/007590**

(87) International publication number:
**WO 2022/265131 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.06.2021 KR 20210077875**

(71) Applicant: **Happymind Co., Ltd**
**Seoul 03908 (KR)**

(72) Inventors:
• YOO, Han Ik
  Seoul 05119 (KR)
• KANG, Sung Hee
  Gwacheon-si, Gyeonggi-do 13833 (KR)

(74) Representative: **Zacco Sweden AB**
**P.O. Box 5581**
**Löjtnantsgatan 21**
**114 85 Stockholm (SE)**

(54) **SYSTEM AND METHOD FOR DIAGNOSING MENTAL DISORDER AND PREDICTING TREATMENT RESPONSE ON BASIS OF PSYCHIATRIC EXAMINATION DATA USING EYE TRACKING**

(57)    Disclosed is a system and method for diagnosing a mental disorder and predicting a treatment response on the basis of psychiatric examination data using eye tracking. The method of diagnosing mental disorders and predicting treatment responses performed by the psychiatric examination system includes generating user attention information using user's eye-tracking on a monitor screen on which a test is being performed, receiving an input of psychiatric examination data including the generated user attention information into a learning model for diagnosing mental disorders and predicting treatment responses, and deriving result information related to mental disorder diagnosis and treatment response from the psychiatric examination data including the user attention information by using the learning model for diagnosing mental disorders and predicting treatment responses.

FIG. 2

START

GENERATE USER ATTENTION INFORMATION USING USER'S EYE TRACKING ON MONITOR SCREEN ON WHICH TEST IS BEING PERFORMED —— S210

RECEIVE INPUT OF PSYCHIATRIC EXAMINATION DATA INCLUDING GENERATED USER ATTENTION INFORMATION INTO LEARNING MODEL FOR DIAGNOSING MENTAL DISORDERS AND PREDICTING TREATMENT RESPONSES —— S220

USE LEARNING MODEL FOR DIAGNOSING MENTAL DISORDERS AND PREDICTING TREATMENT RESPONSES TO DERIVE RESULT INFORMATION RELATED TO MENTAL DISORDER DIAGNOSIS AND TREATMENT RESPONSE FROM PSYCHIATRIC EXAMINATION DATA INCLUDING USER ATTENTION INFORMATION —— S230

END

**Description**

TECHNICAL FIELD

The following description relates to a technology for diagnosing mental disorders and predicting treatment responses on the basis of psychiatric examination data using eye-tracking technology.

BACKGROUND ART

The number of people who experience brain disorders, especially mental disorders expressed by abnormalities in the brain, is gradually increasing. According to the "Mental Illness Survey" conducted by the Ministry of Health and Welfare, as of 2016, out of 5,102 adults aged 18 and over nationwide surveyed, the percentage of people who had suffered from one or more of the 17 major mental illnesses was 25.4%, and the percentage of people who had experienced mental health problems in the past year was 11.9%.

As described above, brain disorders not only seriously interfere with a patient's social life, but also cause serious problems such as suicide when they progress untreated for a long time, and thus need to be managed both personally and socially. However, the number of patients, who do not visit medical institutions because they do not know whether they are suffering from a brain disorder or do not recognize the seriousness of the brain disorder even when they do recognize that they are suffering from the brain disorder, is greater than the number of patients who visit medical institutions and are treated.

As an example, an attention deficit and hyperactivity disorder, which is a condition more commonly known as ADHD, is one of the mental disorders that mainly manifests in childhood. ADHD symptoms are characterized by a persistent lack of attention, leading to distractibility, hyperactivity, and impulsivity, and left untreated, these symptoms may result in difficulties that persist across various behaviors throughout childhood and, in some cases, into adolescence and adulthood. There are cases in which medications have been developed and even patented for children with ADHD symptoms, but the reality is that a complete cure is impossible with the prescription of therapeutic drugs alone, and a certain amount of ongoing practice and training is required.

When ADHD symptoms are suspected or concerned, these ADHD symptoms need to be diagnosed, but it is insufficient to just observe certain behaviors in a doctor's office. Accordingly, there is a need for a technology for diagnosing ADHD and predicting treatment responses through a comprehensive attention test including a (visual) simple selective attention test, a (auditory) simple selective attention test, an inhibition-sustained attention test, an interference-selective attention test, a divided attention test, and a working memory test.

DESCRIPTION OF EMBODIMENTS

TECHNICAL PROBLEM

The present disclosure is directed to providing a method and system for diagnosing mental disorders based on a diagnosis made by a specialist utilizing psychiatric test results including user attention information generated using eye tracking.

TECHNICAL SOLUTION TO PROBLEM

A method of diagnosing mental disorders and predicting treatment responses performed by a psychiatric examination system may include generating user attention information using user's eye-tracking on a monitor screen on which a test is being performed, receiving an input of psychiatric examination data including the generated user attention information into a learning model for diagnosing mental disorders and predicting treatment responses, and deriving result information related to mental disorder diagnosis and treatment response from the psychiatric examination data including the user attention information by using the learning model for diagnosing mental disorders and predicting treatment responses.

The learning model may be constructed by training using a data set for diagnosing mental disorders and predicting treatment responses.

The generating of the user attention information may include determining, from gaze coordinate values using eye tracking, a degree of attention on whether a user is looking at the monitor screen on which the test is being performed or a target area set within the monitor screen on the basis of a pre-set number of frames per second.

The generating of the user attention information may include setting an area of interest in a target area within the monitor screen on which the test is being performed, and determining whether the user is focusing on the set area of interest on the basis of pre-set criteria information.

The generating of the user attention information may include determining eye movement state information related to user's eye movement on a target area within the monitor screen on which the test is being performed using the number of saccades or eye movement fixation.

The generating of the user attention information may include calculating speed information on user's eye movement by using gaze coordinates of a user's gaze on the monitor screen on which the test is being performed or a target area set within the monitor screen and time data, and measuring a variation of the calculated speed information on the user's eye movement.

The generating of the user attention information may include setting the remaining areas other than an area of interest set in a target area within the monitor screen on which the test is being performed as non-interest areas, and determining response inhibition information related to a user's gaze at the set non-interest areas using visual indicators.

The generating of the user attention information may include measuring latency time data for a time until user's eye movement occurs in order to gaze at a new stimulus when the new stimulus appears on the monitor screen on which the test is being performed, in a case in which latency time data on a time until the user's eye movement occurs is greater than or equal to pre-set criteria.

The generating of the user attention information may include extracting data on a change in pupil size of a user according to: an area of interest which is set in a target area within the monitor screen on which the test is being performed; and time.

A psychiatric examination system may include an information generation unit configured to generate user attention information using user's eye tracking on a monitor screen on which a test is being performed, an input unit configured to receive an input of psychiatric examination data including the generated user attention information into a learning model for diagnosing mental disorders and predicting treatment responses, and a result-deriving unit configured to derive result information related to mental disorder diagnosis and treatment response from the psychiatric examination data including the user attention information by using the learning model for diagnosing mental disorders and predicting treatment responses.

ADVANTAGEOUS EFFECTS OF DISCLOSURE

By using eye-tracking technology to derive user attention information, the reliability of a machine learning model for diagnosing mental disorders and predicting treatment responses can be improved.

User attention information extracted using eye-tracking technology can be used to improve result information related to a mental disorder diagnosis and treatment response derived from a machine learning model.

Mental disorders can be more accurately and comprehensively determined through a machine learning model that diagnoses the mental disorders and predicts treatment responses on the basis of a diagnosis made by a specialist utilizing psychiatric examination results. This can be used not only in specialized hospitals but also in psychological counseling centers and the like.

DESCRIPTION OF DRAWINGS

FIG. 1 is a block diagram illustrating a configuration of a psychiatric examination system according to an embodiment.
FIG 2 is a flowchart for describing a method of diagnosing attention deficit and mental disorders and predicting treatment responses in the psychiatric examination system according to an embodiment.
FIGS. 3 and 4 are diagrams for describing deriving result information related to a mental disorder diagnosis and treatment response through a learning model in the psychiatric examination system according to an embodiment.

MODE OF DISCLOSURE

Hereinafter, an embodiment will be described in detail with reference to the accompanying drawings.

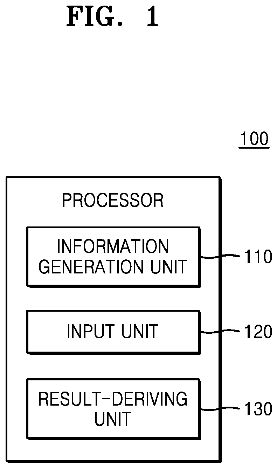

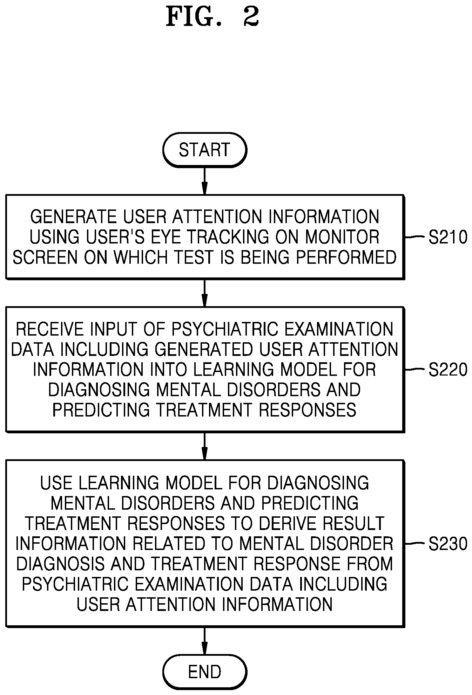

FIG. 1 is a block diagram for describing a configuration of a psychiatric examination system according to an embodiment, and FIG. 2 is a flowchart for describing a method of diagnosing mental disorders and predicting treatment responses in the psychiatric examination system according to an embodiment.

A processor of a psychiatric examination system 100 may include an information generation unit 110, an input unit 120, and a result-deriving unit 130. The components of the processor may be representations of different functions performed by the processor in response to a control instruction provided from a program code stored in the psychiatric examination system. The processor and the components of the processor may control the psychiatric examination system to perform operations 210 to 230 included in the method of diagnosing mental disorders and predicting treatment responses of FIG. 2. Here, the processor and the components of the processor may be implemented to execute an instruction according to a code of at least one program and a code of an operating system (OS) included in a memory.

The processor may load, to the memory, a program code stored in a file of a program for the method of diagnosing mental disorders and predicting treatment responses. For example, when the program is executed in the psychiatric examination system, the processor may control the psychiatric examination system to load the program code from the file of the program to the memory under the control of the OS. Here, the information generation unit 110, the input unit 120, and the result-deriving unit 130 included in the processor may be different functional representations of the processor to perform operations 210 to 230 to be described below by executing an instruction of a portion corresponding to the program code loaded to the memory.

In operation 210, the information generation unit 110 may generate user attention information using user's eye tracking on a monitor screen on which a test is being performed. For example, the information generation unit 110 may generate the user attention information while (comprehensive attention) tests, such as a simple selective attention test, an interference-selective attention test, an inhibition-sustained attention test, a divided attention test, and a working memory test, are performed online in different ways. The information generation unit 110 may generate the user attention information from seven types of data, including a screen state in which the user cannot concentrate on the screen, a visual fixation in which the user cannot focus on the target, an eye movement state with a lot of eye movement, a case in which there is a variation in the speed of eye movement, a response inhibition in which the user sees a target that should not be looked at, a case in which a latency until the eye moves is long, and a case in which a pupil size is changed.

In an example, the information generation unit 110 may determine, from gaze coordinate values using eye tracking, the degree of attention on whether the user is looking at the monitor screen on which the test is being performed or a target area set within the monitor screen on the basis of a pre-set number of frames per second. The number of frames per second indicates a speed at which a film frame (screen) is changed in seconds. For example, in an NTSC (National Television System Committee)-type television, an interlaced scanning method is adopted, and thus, two fields become one frame, and the number of frames per second (FPS) is 30 frames. For example, the information generation unit 110 may obtain a gaze coordinate value of a gaze of the user looking at the monitor screen on which the test is being performed or a target area set within the monitor screen. In this case, the gaze coordinate value may be obtained whenever position data on a position at which the user gazes changes, and the gaze coordinate value (e.g., value of x and y coordinates) may be obtained in real time or at a preset period of time. Using the obtained gaze coordinate values, the information generation unit 110 may measure whether the user is looking at the monitor screen on which the test is being performed or a target area set within the monitor screen by using 30 FPS.

Further, the information generation unit 110 may set an area of interest in the target area within the monitor screen on which the test is being performed, and determine whether the user is focusing on the set area of interest on the basis of pre-set criteria information. In this case, the pre-set criteria information may refer to fixation-related metrics. For example, the information generation unit 110 may obtain a focus score based on whether the user focuses on the area of interest for a preset period of time. The information generation unit 110 may count one point when the user is focusing on the area of interest and minus (-) one point when the user is not focusing. The information generation unit 110 compares the counted focus score with the pre-set criteria information, and when the counted focus score is below the pre-set criteria information as a result of the comparison, the information generation unit 110 may determine that the user is not focusing on the area of interest. Alternatively, the information generation unit 110 may obtain times when the user is focusing or not focusing on the area of interest, and calculate an average time over the obtained times. The information generation unit 110 compares the calculated average time (the average time of not focusing) with the pre-set criteria information, and determines that the user is not focusing on the area of interest when the calculated average time is below the pre-set criteria information as a result of the comparison. In an embodiment, the determination of whether the user is focusing on the area of interest has been illustrated using the count of the focus score and the average time as examples, but various other methods may exist for determining that the user is not focusing on the area of interest. In this case, whether the user is focusing on the area of interest may be measured by only adding simple functions at a program level, utilizing numerical values provided by a software developer kit (SDK) using the fixation-related metrics for the area.

Further, the information generation unit 110 may use the number of saccades or eye movement fixation to determine information on the state of user's eye movement on the target area within the monitor screen on which the test is being performed. For example, the information generation unit 110 may use the total number of saccades during a psychiatric examination to measure the relative number of eye movements for each user. The number of eye movements obtained from various users for whom the psychiatric examination is performed may be stored. This may be used to sort the relative ranking of users on the relative number of eye movements. Alternatively, the information generation unit 110 may measure the relative eye movement state for each user by utilizing an average duration of eye movement fixations. When performing a psychiatric examination, a plurality of tests of different types may be performed, and the information generation unit 110 may measure the average duration of eye movement fixations while each test is being performed. The average duration of eye movement fixation obtained from each of various users for whom the psychiatric examination is performed may be stored. This may be used to short the relative ranking of the average duration of eye movement fixations for each user.

Further, the information generation unit 110 may calculate speed information on user's eye movement by using gaze coordinates of the user's gaze on the monitor screen on which the test is being performed or a target area set within the monitor screen and the time data, measure a variation of the calculated speed information on the user's eye movement. In this case, the variation of the speed information on the eye movement may be measured by adding an arithmetic function code.

Further, the information generation unit 110 may set the remaining areas other than the area of interest set in the target area within the monitor screen on which the test is being performed as non-interest areas, and may determine response inhibition information related to a user's gaze at the set non-interest areas using visual indicators. For example, the information generation unit 110 may use a commission error, a response time (a response mean, a standard deviation, and the like), a total time, and the like as the visual indicators for determining the response inhibition information. The information generation unit 110 may measure time data when a target that should not be gazed at by the user or an area including the target is gazed at, and may determine the response inhibition information by comparing the measured time data with the visual indicator. In this case, the measured time data may be processed (e.g., the mean, the standard deviation, and the like are calculated) according to the response inhibition information.

Further, in a case in which latency time data on a time until user's eye movement occurs is greater than or equal to pre-set criteria, when a new stimulus appears on the monitor screen on which the test is being performed, the information generation unit 110 may measure latency time data on a time until the user's eye movement occurs in order to gaze at the new stimulus.

In addition, the information generation unit 110 may extract data on a change in pupil size of the user according to: the area of interest which is set in the target area within the monitor screen on which the test is being performed; and time. For example, the information generation unit 110 may determine that the user's pupil size decreases or increases.

User attention information may be generated from each piece of data obtained using the eye-tracking technology described above. In this case, each piece of obtained data may be generated into one piece of user attention information by performing a specific operation.

In an embodiment, the generated user attention information may be used as an input parameter of a learning model. Alternatively, psychiatric examination data derived using a learning model may be used to diagnose mental disorders and predict treatment responses by adding the user attention information to test results obtained from the psychiatric examination data.

In operation 220, the input unit 120 may receive an input of the psychiatric examination data including the generated user attention information into a learning model for diagnosing mental disorders and predicting treatment responses. Here, the learning model may be constructed by training using a data set for diagnosing mental disorders and predicting treatment responses. For example, the user attention information and a plurality of pieces of comprehensive attention test data (pieces of data on the simple attention test, the interference-selective attention test, the inhibition-sustained attention test, the divided attention test, and the working memory test) may be used as input data of the learning model. In the case of the screen state, a data set may be obtained by measuring whether the user is looking at the area on the monitor screen, on which the test is being performed, using the gaze coordinate value. In addition, in the case of the visual fixation, a data set may be obtained by measuring whether the user is not focusing on the target by setting up an area of interest (AOI) for the visual target area, using fixation-related metrics (fixation count, fixation mean duration, or the like) for the set AOI, and adding simple functions at a program level using numerical values (fixation or not) provided by the SDK. In addition, in the case of the eye movement state, a data set may be obtained by measuring a relative size of eye movement for each user using the total number of saccades or the average duration of the eye movement fixation. In addition, a variation in the eye movement speed may be calculated using the gaze coordinates and the time value, and a data set may be obtained by measuring the calculated speed variation. In addition, in the case of the response inhibition, a data set may be obtained by setting and measuring an AOI for the response inhibition information, and applying commission error, response time (mean, standard deviation, or the like), and the total time as visual indicators to measure the response inhibition. In addition, in the case in which the latency until the eye moves is long, a data set for a saccadic latency and a movement latency may be obtained by measuring a latency time between the appearance of a new stimulus and the occurrence of eye movement to look at the new stimulus. In addition, a data set may be obtained by extracting data on the change in pupil size according to the target and the time. The seven data sets obtained as described above may be used to train the learning model. Such data sets may be obtained by diagnosing mental disorders and predicting treatment responses. The data sets may also be obtained by diagnosing ADHD, oppositional defiant disorder, conduct disorder, dyslexia, dyscalculia, and autism in people ranging in age from preschoolers to adults, and predicting treatment responses

In operation 230, the result-deriving unit 130 may use the learning model for diagnosing mental disorders and predicting treatment responses to derive result information related to clinical aspects and treatment response of the mental disorder diagnosis from the psychiatric examination data including the user attention information The result-deriving unit 130 may derive drug treatment effect result information related to clinical aspects and treatment response of the mental disorder diagnosis from the psychiatric examination data including the user attention information. In this case, since the measure of importance may be different depending on a domain of a material, the importance between the accuracy of prediction (precision) of mental disorders through the learning model or the rate (recall) of mental disorders correctly predicted by the model may be defined by pre-set criteria. The result-deriving unit 130 may derive the drug treatment effect result information related to the clinical aspects and treatment response of the attention deficit and hyperactivity disorder from the comprehensive attention test data including user attention information. For example, in the diagnosis of attention deficit and hyperactivity disorder, the importance between the accuracy of prediction (precision) of the attention deficit and hyperactivity disorder and the rate (recall) of the attention deficit and hyperactivity disorder correctly predicted by the model may be defined by pre-set criteria. In an embodiment, an example is described in which the prediction accuracy and rate of the attention deficit and hyperactivity disorder are defined by a domain expert. First, a confusion matrix for deriving the drug treatment effect result information may be set. The drug treatment effect result information may be derived on the basis of true positives (TP), false positives (FP), true negatives (TN), and false negatives (FN) configured in the set confusion matrix.

Actual

|   |   | (+) | (−) |   |
|---|---|-----|-----|---|
| Predicted | (+) | TP | FP | Precision |
|  | (−) | FN | TN |  |
|  |  | Recall | Specificity |  |

Accuracy of attention deficit and hyperactivity disorder: $\dfrac{TP+TN}{TP+FP+FN+TN}$

Precision of attention deficit and hyperactivity disorder: $\dfrac{TP}{TP+FP}$

Recall, sensitivity, or True Positive Rate (TPR) of attention deficit and hyperactivity disorder: $\dfrac{TP}{TP+FN}$

Specificity or True Negative Rate (TNR) of attention deficit and hyperactivity disorder: $\dfrac{TN}{TN+FP}$

Here, the accuracy of the attention deficit and hyperactivity disorder means the rate of correct prediction of the attention deficit and hyperactivity disorder, the recall of attention deficit and hyperactivity disorder means the rate of positive samples that are correctly detected, the precision of attention deficit and hyperactivity disorder means the accuracy of positive predictions, and the specificity of attention deficit and hyperactivity disorder means the rate of negative samples correctly detected.

The result-deriving unit 130 may numerically represent a likelihood of a mental disorder. The result-deriving unit 130 can represent the likelihood of a mental disorder as %. For example, the result-deriving unit 130 may express the likelihood of a mental disorder as a numerical value between 0 and 100. In this case, the closer the likelihood of a mental disorder is to 100, the more severe the mental disorder may be determined to be. Alternatively, the result-deriving unit 130 may classify the likelihood of a mental disorder into pre-set classes (e.g., 0 to 25: normal, 25 to 50: slightly at risk, 50 to 75: risk, and 75 to 100: warning), and may derive the class, to which the numerical data of the mental disorder corresponds, on the basis of the classified pre-set classes. The numerical value described in the embodiment is merely an example, and the present disclosure is not limited thereto.

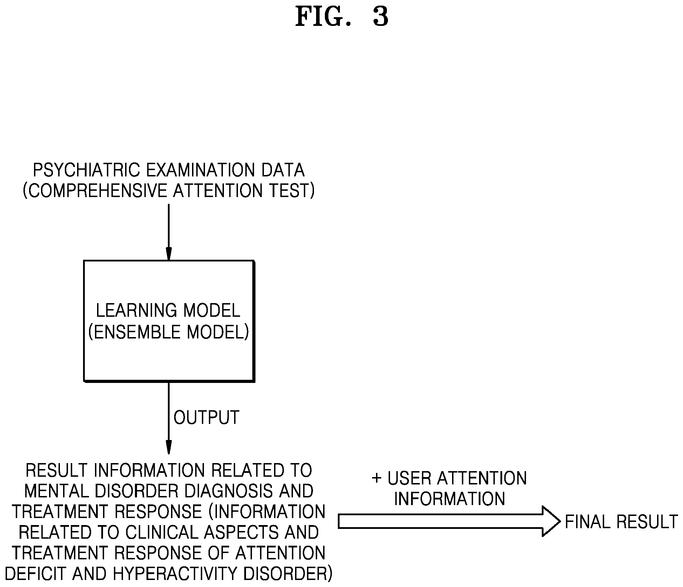

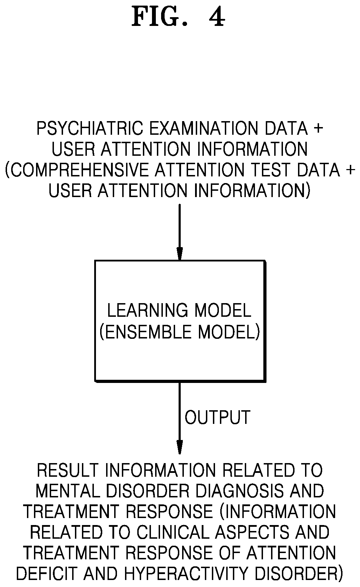

FIGS. 3 and 4 are diagrams for describing deriving result information related to a mental disorder diagnosis and treatment response through a learning model in the psychiatric examination system according to an embodiment.

In FIGS. 3 and 4, a comprehensive attention test (CAT) during a psychiatric examination will be described as an example. In addition, numerical values described in the specification are merely examples, and the present disclosure is not limited thereto.

The term "comprehensive attention test" refers to a standardized test tool that comprehensively evaluates attention in children, adolescents, and adults. The term "attention" refers to higher functions of the brain that enable individuals to focus on desired information, sustain concentration for problem-solving, and sometimes redirect attention in response to required stimuli according to a purpose. The comprehensive attention test may examine five different types of attention, including simple selective attention, interference-selective attention, inhibition-sustained attention, divided attention, and working memory, through six different tests. Through this, each level of attention may be identified, and attention deficits may be evaluated.

The (visual) simple selective attention refers to the ability to respond to a desired visual stimulation, the (auditory) simple selective attention refers to the ability to respond to a desired auditory stimulation, the inhibition-sustained attention refers to the ability to continuously maintain attention and inhibit impulsivity, the interference-selective attention refers to the ability to ignore an interferential stimulation and respond to a required stimulation, the divided attention refers to the ability to simultaneously process two or more stimuli, and the working memory refers to the ability to remember and process a sequence of stimuli in order.

In an example, in the comprehensive attention test, a menu of age-specific default sets may exist, and individual tests may be combined considering the situation of an examiner to create a new set of tests suitable for the examiner. For example, a kindergarten set for ages 4 to 5 may consist of (visual and auditory: each 10 minutes) simple selective attention tests and an inhibition-sustained attention test, a shortened kindergarten set (for ages 4 to 5) may consist of (visual and auditory: each 5 minutes) simple selective attention tests and the inhibition-sustained attention test, a lower grade set for ages 6 to 8 may consist of the (visual and auditory: each 10 minutes) simple selective attention tests, the inhibition-sustained attention test, and an interference-selective attention test, a shortened lower grade set (for ages 6 to 8) may consist of the (visual and auditory: each 5 minutes) simple selective attention tests, the inhibition-sustained attention test, and the interference-selective attention test, a higher grade set for ages 9 to 15/adult set for ages 16 and older may consist of the inhibition-sustained attention test, the interference-selective attention test, a divided attention test, and a working memory test, and a comprehensive test set for ages 9 to 15/ comprehensive test set for ages 16 and older may consist of the (visual and auditory: each 10 minutes) simple selective attention tests, the inhibition-sustained attention test, the interference-selective attention test, the divided attention test, and the working memory test.

The psychiatric examination system may include comprehensive attention test data related to the comprehensive attention test results. Here, the comprehensive attention test data may include hospital data collected from each hospital and standardized data obtained by standardizing values of the hospital data. For example, the comprehensive attention test data may include hospital data including diagnosis made by a specialist using the hospital data and the comprehensive attention test data. In addition, standardization may be performed to use only a specific range of data from each hospital data, to unify units of the test data, or to reflect the fact that different hospitals have different diagnostic criteria.

The psychiatric examination system may perform analysis on the comprehensive attention test data. The psychiatric examination system may set variables to remove and exclude a plurality of types of outliers from the comprehensive attention test data. For example, five types of outliers may be set. The five types of outliers may include type 1, characterized by ages falling outside a range of fewer than 4 years or more than 100 years, type 2, characterized by cases where test parameters for either visual or auditory simple selective attention are omitted, type 3, characterized by cases where the number of correct responses and a spatial attention width in a reverse direction for the working memory test are omitted, type 4, characterized by cases where working memory observations, as well as the spatial attention width in the reverse direction and an attention quotient (AQ) for the working memory test, are omitted, and type 5 characterized by cases where gender information is not provided or included. The psychiatric examination system may remove at least one or more and/or all of the five types of outliers from the comprehensive attention test data. In addition, the psychiatric examination system may set variables for hospital, magnetic resonance (MR), co-occurring diagnoses, severity, treatment status, and treatment response, which are selected from the comprehensive attention test data and are utilized as exclusion variables.

The psychiatric examination system may selectively select test data representative of each test from the comprehensive attention test data on the basis of predefined criteria. In other words, the psychiatric examination system may exclude some test data from modeling for the comprehensive attention test data on the basis of the predefined criteria. As a result of the exclusion, a degree of imbalance between normal test data and ADHD test data can be improved compared to before the exclusion. Here, resampling methods for improving the degree of imbalance, e.g., a synthetic minority over-sampling technique (SMOTE), random oversampling, are not considered. SMOTE is a method of randomly generating similar data for each class, and may generate random data by simply filling a section between each point in the same class. The psychiatric examination system may perform under-sampling to exclude ADHD data and normal data of the hospital data, when the ADHD data is within the range of the standardized data or the normal data is outside the range of the standardized data.

Standardization may be performed to unify units for the test data (values) obtained by performing the comprehensive attention test. For example, in the case of hospital data, "Scaling with Random Over-Sampling" may be performed as a standardization method. In "Scaling with Random Over-Sampling," "StandardScaler," "RobustScaler," and "Min-MaxScaler" may be performed on the comprehensive attention test data.

Referring to FIG. 3, the psychiatric examination system may perform modeling for generating a learning model by selectively selecting test data related to the comprehensive attention test, and may classify attention deficit and hyperactivity disorder and predict treatment response through the learning model.

In an example, the psychiatric examination system may generate a random forest-based ensemble model as a learning model to train the comprehensive attention test data. The psychiatric examination system may input the comprehensive attention test data into the learning model. The psychiatric examination system may classify the comprehensive attention test data for each test. For example, the psychiatric examination system may classify the comprehensive attention test data into simple attention, interference-selective attention, inhibition-sustained attention, divided attention, and working memory.

The psychiatric examination system may perform pre-processing on each test data classified for each test. The psychiatric examination system may classify each classification model for the pre-processed test data by using random forest. The psychiatric examination system may estimate a missing value for each test data classified using the random forest. In this case, specific processing may be performed on the missing value. For example, specific processing may be performed, such as deleting the missing value or replacing the missing value with another value.

After the missing value is processed, the psychiatric examination system may derive the likelihood of the attention deficit and hyperactivity disorder by classifying each test data classified using the random forest using random forest.

In another example, the psychiatric examination system may train comprehensive attention test data by generating an ensemble model based on a Gaussian process classifier as the learning model. The psychiatric examination system may input the comprehensive attention test data into the learning model. The psychiatric examination system may classify the comprehensive attention test data for each test. For example, the psychiatric examination system may classify the comprehensive attention test data into simple attention, interference-selective attention, inhibition-sustained attention, divided attention, and working memory. The psychiatric examination system may perform a range test on the standardized data for each test data classified for each test. In this case, the range test for the working memory is not performed.

As the test proceeds, the psychiatric examination system may merge each test data that has passed the test. At this point, after all the test data are merged, the psychiatric examination system may exclude test data for which at least three of the simple attention test, the interference-selective attention test, the inhibition-sustained attention test, and the divided attention test are not performed.

The psychiatric examination system may estimate the missing value by excluding test data for which three or more of the simple attention test, the interference-selective attention test, the inhibition-sustained attention test, and the divided attention test are not performed. For example, when a subject did not take the (visual) simple selective attention test and took the (auditory) simple selective attention test, the inhibition-sustained attention test, the interference-selective attention test, and the divided attention test, the psychiatric examination system may estimate the (visual) simple selective attention test data by averaging test data for each of the (auditory) simple selective attention, the inhibition-sustained attention, the interference-selective attention, and the divided attention. In this case, specific processing may be performed on the estimated missing value. For example, specific processing may be performed, such as deleting the missing value or replacing the missing value with another value.

The psychiatric examination system may reduce high-dimensional data to low-dimensional data through principal component analysis (PCA) since measurement values of all the test data are merged. Since the number of columns becomes approximately 100 or more as the measurement values of all the test data are merged, the psychiatric examination system can use the PCA to reduce the dimensions to about 30. The psychiatric examination system may replace the classifier with a Gaussian process classifier. The psychiatric examination system may classify the merged test data whose dimension is reduced using the Gaussian process classifier to classify the attention deficit and hyperactivity disorder and derive the likelihood of the treatment response.

The psychiatric examination system according to an embodiment may use the Gaussian process classifier as a classifier to enhance the performance of the learning model that simultaneously utilizes test data for the simple attention, the interference-selective attention, the inhibition-sustained attention, the divided attention, and the working memory that are included in the comprehensive attention test data. Accordingly, a reasonable probability of attention deficit and hyperactivity disorder is attained. However, when the prevalence rate is assumed to be 10%, the precision of the learning model decreases. When the prevalence rate is assumed to be 10%, a learning model may be used, but the learning model may be validated by sampling a class balance of test data, which is not used for modeling, to be 10% for the attention deficit and hyperactivity disorder and 90% for the normal. Since the ratio of the attention deficit and hyperactivity disorder to the normal is set to 10: 90, rather than taking into account actual symptom extremes or differences from the normal, the absolute number of the attention deficit and hyperactivity disorder reduced and an accuracy value is lowered when the prevalence rate is assumed to be 10%.

As described above, the psychiatric examination system may derive result information related to clinical aspects and treatment responses of the attention deficit and hyperactivity disorder for the comprehensive attention test data by using the learning model. For example, the psychiatric examination system may classify the attention deficit and hyperactivity disorder and derive the likelihood of treatment response. The psychiatric examination system may obtain a final result by applying the user attention information to the derived test result. The psychiatric examination system may obtain the final result by extracting valid data from the derived test result by using eye-tracking technology. The psychiatric examination system may generate the user attention information in relation to attention level determination, whether the area of interest is focused, eye movement state information on eye movement, speed/velocity variation of eye movements, response inhibition information when gazing at a target that should not be looked at, latency time data until eye movement occurs, data about change in pupil size, and the like.

Specifically, the psychiatric examination system may determine, from the gaze coordinate values using eye tracking, the degree of attention on whether the user is looking at the monitor screen on which the test is being performed or a target area set within the monitor screen on the basis of the pre-set number of frames per second. Further, the psychiatric examination system may set an area of interest in the target area within the monitor screen on which the test is being performed, and determine whether the user is focusing on the set area of interest on the basis of pre-set criteria information. Further, the psychiatric examination system may use the number of saccades or eye movement fixation to determine information on the state of user's eye movement on the target area within the monitor screen on which the test is being performed. Alternatively, the psychiatric examination system may measure the relative eye movement state for each user by utilizing an average duration of eye movement fixations. Further, the psychiatric examination system may calculate speed information on user's eye movement by using gaze coordinates of the user's gaze on the monitor screen on which the test is being performed or a target area set within the monitor screen and the time data, measure a variation of the calculated speed information on the user's eye movement. Further, the psychiatric examination system may set the remaining areas other than the area of interest set in the target area within the monitor screen on which the test is being performed as non-interest areas, and may determine response inhibition information related to a user's gaze at the set non-interest areas using visual indicators. Further, in a case in which latency time data on a time until user's eye movement occurs is greater than or equal to pre-set criteria, when a new stimulus appears on the monitor screen on which the test is being performed, the psychiatric examination system may measure latency time data on a time until the user's eye movement occurs in order to gaze at the new stimulus. In addition, the psychiatric examination system may extract data on a change in pupil size of the user according to: the area of interest which is set in the target area within the monitor screen on which the test is being performed; and time.

The psychiatric examination system may obtain a more accurate final result by applying the user attention information generated using eye-tracking technology to the derived test result. According to an embodiment, it is possible to predict diagnosis and treatment response in ADHD, oppositional defiant disorder, conduct disorder, dyslexia, dyscalculia, and even autism through the comprehensive attention test.

Referring to FIG. 4, the psychiatric examination system may selectively select test data related to the comprehensive attention test. The psychiatric examination system may use the selected test data and user attention information to perform modeling for generating a learning model, and the learning model may be used to classify attention deficit and hyperactivity disorder and predict treatment response. In an example, an ensemble model based on a random forest may be used as the learning model. Here, the learning model may be constructed by training using a data set in advance. The psychiatric examination system may input the user attention information and the comprehensive attention test data to the learning model. The psychiatric examination system may classify the user attention information and the comprehensive attention test data on the basis of the pre-set criteria (e.g., type of test). For example, the psychiatric examination system may classify the comprehensive attention test data into simple attention, interference-selective attention, inhibition-sustained attention, divided attention, and working memory.

The psychiatric examination system may perform pre-processing on each test data classified for each test and the user attention information. The psychiatric examination system may classify each classification model for the pre-processed test data and the user attention information by using random forest. The psychiatric examination system may estimate a missing value for each of the test data and the user attention information classified using the random forest. In this case, specific processing may be performed on the missing value. For example, specific processing may be performed, such as deleting the missing value or replacing the missing value with another value.

After the missing value is processed, the psychiatric examination system may derive the likelihood of attention deficit and hyperactivity disorder by classifying each of the test data and the user attention information classified using the random forest using random forest.

In another example, the psychiatric examination system may use a Gaussian process classifier-based ensemble model as the learning model. Here, the learning model may be constructed by training by using a data set in advance. The psychiatric examination system may input the comprehensive attention test data and the user attention information into the learning model. The psychiatric examination system may classify the comprehensive attention test data and the user attention information on the basis of the pre-set criteria (e.g., type of test) For example, the psychiatric examination system may classify the comprehensive attention test data into simple attention, interference-selective attention, inhibition-sustained attention, divided attention, and working memory. The psychiatric examination system may perform a range test on the standardized data for each of the test data classified for each test and the user attention information. At this time, a range test for the working memory is not performed.

As the test proceeds, the psychiatric examination system may merge each of the test data and the user attention information that have passed the test. At this point, after all of the test data and the user attention information are merged, the psychiatric examination system may exclude test data for which at least three of the simple attention test, the interference-selective attention test, the inhibition-sustained attention test, and the divided attention test were not performed.

The psychiatric examination system may estimate the missing value by excluding test data for which three or more of the simple attention test, the interference-selective attention test, the inhibition-sustained attention test, and the divided attention test are not performed. For example, when a subject did not take the (visual) simple selective attention test and took the (auditory) simple selective attention test, the inhibition-sustained attention test, the interference-selective attention test, and the divided attention test, the comprehensive attention test system may estimate the (visual) simple selective attention test data by averaging test data for each of the (auditory) simple selective attention, the inhibition-sustained attention, the interference-selective attention, and the divided attention. In this case, specific processing may be performed on the estimated missing value. For example, specific processing may be performed, such as deleting the missing value or replacing the missing value with another value.

The psychiatric examination system may reduce high-dimensional data to low-dimensional data through principal component analysis (PCA) since measurement values of all of the test data and the user attention information are merged. Since the number of columns becomes approximately 100 or more as the measurement values of all of the test data and the user attention information are merged, the psychiatric examination system can use the PCA to reduce the dimensions to about 30. The psychiatric examination system may replace the classifier with a Gaussian process classifier. The psychiatric examination system may classify the merged test data whose dimension is reduced using the Gaussian process classifier to classify the attention deficit and hyperactivity disorder and derive the likelihood of the treatment response.

## MODES FOR CARRYING OUT THE INVENTION

The device described above may be implemented in hardware components, software components, and/or a combination of the hardware components and software components. For example, the device and components described in embodiments may be implemented using one or more general purpose computers or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of executing and responding to instructions. A processing device may execute an operating system (OS) and one or more software applications running on the operating system. Further, the processing device may access, store, manipulate, process, and generate data in response to execution of the software. For ease of understanding, it has been described that a single processing device is used in some cases, but those skilled in the art will appreciate that the processing device may include a plurality of processing elements and/or a plurality of types of processing elements. For example, the processing device may include a plurality of processors, or a single processor and a single controller. Further, other processing configurations such as a parallel processor are possible.

The software may include a computer program, a code, an instruction, or a combination of one or more thereof, configure the processing device to operate as desired, or independently or collectively command the processing device to operate as desired. The software and/or data may be permanently or temporarily embodied in any type of a machine, a component, a physical device, virtual equipment, a computer storage medium or device, or a signal wave which will be transmitted in order to be interpreted by the processing device or provide a command or data to the processing device. The software may be distributed over computer systems connected through a network and be stored or executed in a distributed manner. The software and data may be stored in one or more computer-readable recording media.

The method according to the embodiment may be implemented in the form of a program instruction which is executable through various computer means and be recorded in a computer-readable medium. The computer-readable medium may include program instructions, data files, data structures, and the like alone or in a combination thereof. The program instructions recorded on the media may be those specially designed and configured for embodiments or may be known and available to those skilled in the art of computer software. Examples of the computer-readable recording medium include magnetic media such as a hard disk, a floppy disk, and a magnetic tape, optical media such as CD-ROM and DVD, magneto-optical media such as floptical disks, and hardware devices specially configured to store and perform program commands such as such as read-only memory (ROM), random-access memory (RAM), and flash memory. Examples of the program instructions may include not only machine codes created by a compiler, but also high-level language codes that may be executed by a computer using an interpreter and the like.

Although the embodiments have been described above with reference to the limited embodiments and drawings, those skilled in the art may apply various technical modifications and variations on the basis of the above description. For example, appropriate results may be achieved even when the described technique is performed in a different order from the described method and/or the components of the described system, structure, device, circuit and the like may be combined in a different form or replaced or substituted by other components or equivalents.

Therefore, other implementations, other embodiments, and those equivalents to the claims are within the scope of the claims to be described below.

## Claims

1. A method of diagnosing mental disorders and predicting treatment responses performed by a psychiatric examination system, the method comprising:

   generating user attention information using user's eye tracking on a monitor screen on which a test is being performed;
   receiving an input of psychiatric examination data including the generated user attention information into a learning model for diagnosing mental disorders and predicting treatment responses; and
   deriving result information related to mental disorder diagnosis and treatment response from the psychiatric examination data including the user attention information by using the learning model for diagnosing mental disorders and predicting treatment responses.

2. The method of claim 1, wherein the learning model is constructed by training using a data set for diagnosing mental disorders and predicting treatment responses.

3. The method of claim 1, wherein the generating of the user attention information includes determining, from gaze coordinate values using eye tracking, a degree of attention on whether a user is looking at the monitor screen on which the test is being performed or a target area set within the monitor screen on the basis of a pre-set number of frames per second.

4. The method of claim 1, wherein the generating of the user attention information includes setting an area of interest in a target area within the monitor screen on which the test is being performed, and determining whether the user is focusing on the set area of interest on the basis of pre-set criteria information.

5. The method of claim 1, wherein the generating of the user attention information includes determining eye movement state information related to user's eye movement on a target area within the monitor screen on which the test is being performed using the number of saccades or eye movement fixation.

6. The method of claim 1, wherein the generating of the user attention information includes calculating speed information on user's eye movement by using gaze coordinates of a user's gaze on the monitor screen on which the test is being performed or a target area set within the monitor screen and time data, and measuring a variation of the calculated speed information on the user's eye movement.

7. The method of claim 1, wherein the generating of the user attention information includes setting the remaining areas other than an area of interest set in a target area within the monitor screen on which the test is being performed as non-interest areas, and determining response inhibition information related to a user's gaze at the set non-interest areas using visual indicators.

8. The method of claim 1, wherein the generating of the user attention information includes measuring latency time data for a time until user's eye movement occurs in order to gaze at a new stimulus when the new stimulus appears on the monitor screen on which the test is being performed, in a case in which latency time data on a time until the user's eye movement occurs is greater than or equal to pre-set criteria.

9. The method of claim 1, wherein the generating of the user attention information includes extracting data on a change in pupil size of a user according to:
   an area of interest which is set in a target area within the monitor screen on which the test is being performed; and time.

10. A psychiatric examination system comprising:

an information generation unit configured to generate user attention information using user's eye tracking on a monitor screen on which a test is being performed;

an input unit configured to receive an input of psychiatric examination data including the generated user attention information into a learning model for diagnosing mental disorders and predicting treatment responses; and

a result-deriving unit configured to derive result information related to mental disorder diagnosis and treatment response from the psychiatric examination data including the user attention information by using the learning model for diagnosing mental disorders and predicting treatment responses.

# FIG. 1

100

PROCESSOR

| INFORMATION GENERATION UNIT | 110 |

| INPUT UNIT | 120 |

| RESULT-DERIVING UNIT | 130 |

# FIG. 2

START

GENERATE USER ATTENTION INFORMATION USING USER'S EYE TRACKING ON MONITOR SCREEN ON WHICH TEST IS BEING PERFORMED — S210

RECEIVE INPUT OF PSYCHIATRIC EXAMINATION DATA INCLUDING GENERATED USER ATTENTION INFORMATION INTO LEARNING MODEL FOR DIAGNOSING MENTAL DISORDERS AND PREDICTING TREATMENT RESPONSES — S220

USE LEARNING MODEL FOR DIAGNOSING MENTAL DISORDERS AND PREDICTING TREATMENT RESPONSES TO DERIVE RESULT INFORMATION RELATED TO MENTAL DISORDER DIAGNOSIS AND TREATMENT RESPONSE FROM PSYCHIATRIC EXAMINATION DATA INCLUDING USER ATTENTION INFORMATION — S230

END

# FIG. 3

PSYCHIATRIC EXAMINATION DATA
(COMPREHENSIVE ATTENTION TEST)

LEARNING MODEL
(ENSEMBLE MODEL)

OUTPUT

RESULT INFORMATION RELATED TO
MENTAL DISORDER DIAGNOSIS AND
TREATMENT RESPONSE (INFORMATION
RELATED TO CLINICAL ASPECTS AND
TREATMENT RESPONSE OF ATTENTION
DEFICIT AND HYPERACTIVITY DISORDER)

+ USER ATTENTION
INFORMATION

FINAL RESULT

# FIG. 4

PSYCHIATRIC EXAMINATION DATA +
USER ATTENTION INFORMATION
(COMPREHENSIVE ATTENTION TEST DATA +
USER ATTENTION INFORMATION)

LEARNING MODEL
(ENSEMBLE MODEL)

OUTPUT

RESULT INFORMATION RELATED TO
MENTAL DISORDER DIAGNOSIS AND
TREATMENT RESPONSE (INFORMATION
RELATED TO CLINICAL ASPECTS AND
TREATMENT RESPONSE OF ATTENTION
DEFICIT AND HYPERACTIVITY DISORDER)

## INTERNATIONAL SEARCH REPORT

| | International application No. |
|---|---|
| | **PCT/KR2021/007590** |

| | |
|---|---|
| **A.** | **CLASSIFICATION OF SUBJECT MATTER** |
| | **A61B 5/16**(2006.01)i; **A61B 5/00**(2006.01)i |
| | According to International Patent Classification (IPC) or to both national classification and IPC |

| | |
|---|---|
| **B.** | **FIELDS SEARCHED** |
| Minimum documentation searched (classification system followed by classification symbols) |
| | A61B 5/16(2006.01); A61B 10/00(2006.01); A61B 3/113(2006.01); A61B 5/00(2006.01); G16H 10/20(2018.01); G16H 50/20(2018.01) |
| Documentation searched other than minimum documentation to the extent that such documents are included in the fields searched |
| | Korean utility models and applications for utility models: IPC as above Japanese utility models and applications for utility models: IPC as above |
| Electronic data base consulted during the international search (name of data base and, where practicable, search terms used) |
| | eKOMPASS (KIPO internal) & keywords: 아이트래킹(eye-tracking), 학습 모델(training model), 정신 장애(mental disorder), 주의력(attention), 예측(prediction) |

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| X | KR 10-2020-0005986 A (DOBRAIN INC.) 17 January 2020 (2020-01-17) See paragraphs [0037]-[0042], [0063], [0103], [0115]-[0126], [0163], [0192]-[0199] and [0214]-[0216]. | 1,2,10 |
| Y | | 3-9 |
| Y | JP 2021-504093 A (VIEWMIND S.A.) 15 February 2021 (2021-02-15) See paragraphs [0018]-[0032], [0042], [0046] and [0074]. | 3-9 |
| A | KR 10-2021-0019266 A (BRAIN&RESEARCH INNOVATION CO., LTD.) 22 February 2021 (2021-02-22) See entire document. | 1-10 |
| A | JP 2015-503414 A (UNIVERSITY COURT OF THE UNIVERSITY OF ABERDEEN) 02 February 2015 (2015-02-02) See entire document. | 1-10 |

| | | | |
|---|---|---|---|
| ☑ Further documents are listed in the continuation of Box C. | | ☑ See patent family annex. | |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 March 2022** | **11 March 2022** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

# EP 4 356 833 A1

**INTERNATIONAL SEARCH REPORT**

International application No.

**PCT/KR2021/007590**

**C.    DOCUMENTS CONSIDERED TO BE RELEVANT**

| Category* | Citation of document, with indication, where appropriate, of the relevant passages | Relevant to claim No. |
|---|---|---|
| A | JP 6865996 B1 (SUSMED, INC.) 28 April 2021 (2021-04-28)<br>See entire document. | 1-10 |

<table>
<tr><td colspan="2" align="center">**INTERNATIONAL SEARCH REPORT**<br>**Information on patent family members**</td><td colspan="2">International application No.<br><br>**PCT/KR2021/007590**</td></tr>
</table>

| Patent document<br>cited in search report | Publication date<br>(day/month/year) | Patent family member(s) | Publication date<br>(day/month/year) |
|---|---|---|---|
| KR 10-2020-0005986 A | 17 January 2020 | KR 10-2020-0005987 A | 17 January 2020 |
|  |  | KR 10-2166010 B1 | 15 October 2020 |
|  |  | KR 10-2166011 B1 | 15 October 2020 |
| JP 2021-504093 A | 15 February 2021 | AR 114151 A1 | 29 July 2020 |
|  |  | CA 3086037 A1 | 06 June 2019 |
|  |  | CN 111970958 A | 20 November 2020 |
|  |  | EP 3716837 A1 | 07 October 2020 |
|  |  | US 2021-0174959 A1 | 10 June 2021 |
|  |  | WO 2019-106678 A1 | 06 June 2019 |
| KR 10-2021-0019266 A | 22 February 2021 | KR 10-2262889 B1 | 09 June 2021 |
| JP 2015-503414 A | 02 February 2015 | AU 2013-207132 A1 | 24 July 2014 |
|  |  | AU 2013-207132 A1 | 11 July 2013 |
|  |  | AU 2013-207132 B2 | 10 May 2018 |
|  |  | CA 2860455 A1 | 11 July 2013 |
|  |  | EP 2800507 A1 | 12 November 2014 |
|  |  | US 2014-0364761 A1 | 11 December 2014 |
|  |  | WO 2013-102768 A1 | 11 July 2013 |
| JP 6865996 B1 | 28 April 2021 | None |  |

Form PCT/ISA/210 (patent family annex) (July 2019)